# EUROPÄISCHE PATENTSCHRIFT

(11) **EP 1 575 667 B1**
(45) Veröffentlichungstag und Bekanntmachung des Hinweises auf die Patenterteilung: **19.09.2007**
(21) Anmeldenummer: 03788999.5
(22) Anmeldetag: 04.11.2003
(51) Int. Cl.: A61N 1/39

(54) **DEFIBRILLATOR**
DEFIBRILLATOR
DEFIBRILLATEUR

(30) Priorität: 05.11.2002 DE 10251344
(43) Veröffentlichungstag der Anmeldung: 21.09.2005
(73) Patentinhaber: Metrax GmbH, D-78628 Rottweil (DE)
(72) Erfinder: BUCHER, Jürgen, 78628 Rottweil (DE)
(74) Vertreter: Fleck, Hermann-Joseph
(86) Internationale Anmeldenummer: PCT/EP2003/012264
(87) Internationale Veröffentlichungsnummer: WO 2004/041360

(56) Entgegenhaltungen:
- WO-A-03/017819
- WO-A-03/024521
- WO-A-03/028799
- WO-A-03/097161
- US-A1- 2003 167 075
- US-B1- 6 263 238

## Beschreibung

Die Erfindung bezieht sich auf einen Defibrillator mit einer in einem Gehäuse (2) angeordneten Elektronik und daran anschließbaren, an einem Patienten anzulegenden Elektroden.

Ein derartiger Defibrillator ist beispielsweise in der EP 0 801 959 A2 gezeigt. Dabei werden Patientenelektroden mittels eines Verbindungskabels an einer auf der Außenseite einer Gehäusewandung angeordneten Buchse angeschlossen. Ferner ist auf einer Außenseite des Gehäuses ein Bedienungsfeld mit Bedien- und Anzeigeelementen vorgesehen. Solche Defibrillatoren werden immer häufiger als Notfallgeräte in öffentlich zugänglichen Gebäuden oder an anderen geeigneten Plätzen positioniert, damit sie im Notfall schnell verfügbar sind. Derartige Defibrillatoren sollen möglichst einfach ausgebildet sein, damit sie auch von einem ungeübten Benutzer bedient werden können.

In der US 5,502,894 sind vakuumdicht verpackte Patientenelektroden angegeben. Dokument WO-A-03/024521 offenbart eine Vorrichtung gemäß Oberbegriff des Anspruchs 1.

Der Erfindung liegt die Aufgabe zugrunde, einen Defibrillator der eingangs genannten Art bereitzustellen, der besonders benutzerfreundlich ausgebildet ist.

Diese Aufgabe wird mit den Merkmalen des Anspruchs 1 gelöst.

Um eine zuverlässige Funktionsfähigkeit auf längere Sicht zu gewährleisten sind weiterhin die Maßnahmen vorteilhaft, dass die Elektroden mittels einer vakuumdichten Elektrodenaufnahme in der Kammer der Abdeckung aufgenommen sind. Hierbei können gleichzeitig auch zyklisch ablaufende Elektrodentestvorgänge programmiert sein, wobei die Elektroden geeignet ausgebildet und angeordnet sind und, wie an sich bekannt, auf ihrer Wirkfläche mit einer Gelschicht versehen sind. Die Elektroden können mit ihrer Aufnahme leicht aus der Kammer der Abdeckung entnommen werden oder aber schon beim Abnehmen der Abdeckung von dem Gehäuse freigegeben werden, indem die Elektrodenaufnahme durch ihre Konstruktion und Anordnung im Gehäuse aufgerissen wird.

Für die Bedienung weitere vorteilhafte Maßnahmen bestehen darin, dass an der Außenseite der Abdeckung ein von einem Benutzer erfassbares Griffmittel angeordnet ist, mit dem die Abdeckung von dem Gehäuse wegreißbar ist.

Dabei besteht eine günstige Ausbildung darin, dass das Griffmittel eine mit der Abdeckung oder mit der Elektrodenaufnahme verbundene Grifflasche ist. Ist die Griffflasche beispielsweise an der Elektrodenaufnahme befestigt, wird sie nach Benutzung des Defibrillators beim Bestücken der Kammer mit einer neuen Elektrodenaufnahme miterneuert, wobei die Grifflasche von der Innenseite der Abdeckung nach außen geführt wird.

Für die Bedienung vorteilhafte Ausgestaltungen bestehen weiterhin darin, dass die Abdeckung mit Haltemitteln versehen ist, die zum Fixieren an dem Gehäuse in an diesem angeordneten Gegenhalteelementen eingerastet, eingeklipst oder eingeschnappt sind. Die Haltemittel und/oder Gegenhalteelemte können dabei vorteilhaft an der Abdeckung bzw. dem Gehäuse angeformt sein.

Zur benutzerfreundlichen Ausbildung trägt ferner bei, dass auf der Innenseite der Abdeckung weitere Kammern ausgebildet und weitere herausnehmbare Bedienungsgegenstände aufgenommen sind. Als Bedienungsgegenstände kommen dabei z.B. ein Rasierapparat, Handschuhe, eine Bürste oder dgl. in Betracht, die bei der Verwendung des Defibrillators von Nutzen sind.

Für eine geordnete Unterbringung und einen eindeutigen Abschluss auf der Innenseite der Abdeckung kann ferner vorteilhaft vorgesehen sein, dass die mindestens eine Kammer mit den Elektroden und gegebenenfalls weiteren Bediengegenständen mittels eines abnehmbaren inneren Abdeckteils abgedeckt ist.

Für die Funktionsfähigkeit und einfache Benutzung sind des Weiteren die Maßnahmen vorteilhaft, dass eine von der Abdeckung im nicht benutzten Zustand abgedeckte und im Benutzungszustand freigegebene frontseitige Gehäusewandung als Bedienfeld mit mindestens einem Auslöseelement für die Defibrillation und Benutzerführungselementen ausgebildet ist.

Einer einfachen Benutzung kommt weiterhin zu Gute, dass auf der Außenseite der Abdeckung ein Informationsmittel zum Betätigen des Griffmittels angeordnet ist. Beispielsweise kann das Informationsmittel ein schnell erfassbares Symbol auf der Außenseite der Abdeckung sein.

Die Erfindung wird nachfolgend anhand eines Ausführungsbeispiels unter Bezugnahme auf die Zeichnungen näher erläutert. Es zeigen:
- Fig. 1: einen Defibrillator mit Halterung in perspektivischer Ansicht,
- Fig. 2: eine Abdeckung des Defibrillators nach Fig. 1 in perspektivischer Ansicht,
- Fig. 3: den Defibrillator nach Fig. 1 bei abgenommener Abdeckung,
- Fig. 4a), b) und c): die Innenseite der Abdeckung nach Fig. 2 in drei verschiedenen Zuständen der Bestückung,
- Fig. 5: einen teilweise geschnittenen Ausschnitt des Defibrillators im Bereich eines Halteabschnitts der Abdeckung,
- Fig. 6: eine weitere perspektivische Darstellung des Defibrillators bei abgenommener Abdeckung und
- Fig. 7a) bis f): verschiedene Ansichten der Abdeckung.

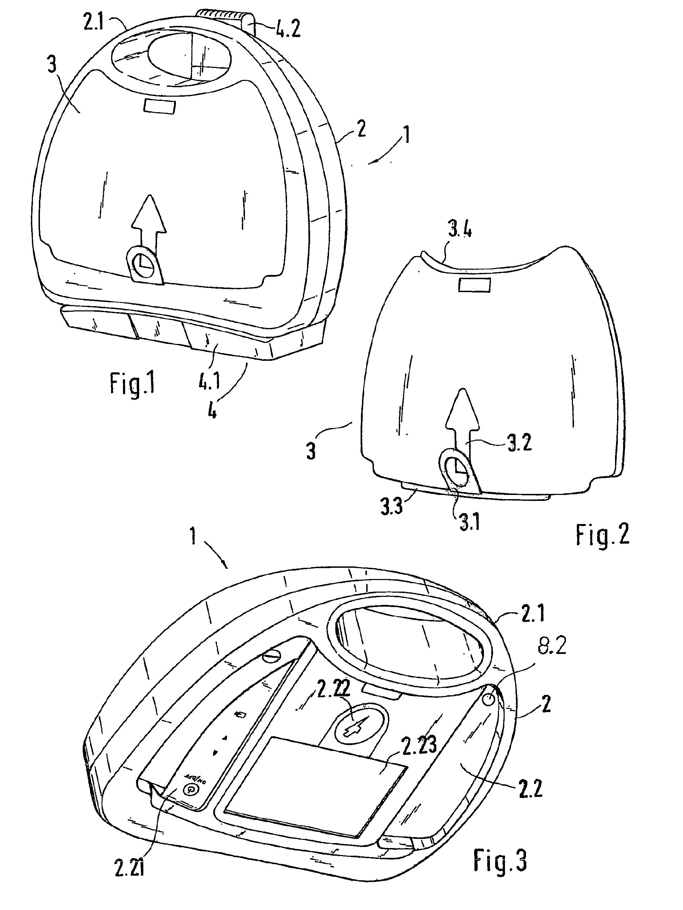

Fig. 1 zeigt einen Defibrillator 1 mit einem Gehäuse 2, in dessen Vorderseite eine Abdeckung 3 eingesetzt ist und der mit einer Halterung 4 mit Fußteil 4.1 und rückseitigem Stützteil oder Halteteil 4.2 versehen ist. Ein derartiger Defibrillator, ein sogenannter automatischer externer Defibrillator (kurz AED), soll im Notfall auch von einem ungeübten Benutzer bedient werden können und ist vorzugsweise an einer gut zugänglichen Stelle positioniert.

Das in seiner äußeren Kontur gerundet ausgestaltete Gehäuse 2 weist in seinem oberen Bereich einen integrierten Griffteil 2.1 auf und besitzt auf seiner Vorderseite eine beispielsweise mit einem Bedienabschnitt 2.21, einem Auslöseelement 2.22 für die Defibrillation und einer Anzeigeeinheit 2.23 versehene Bedienoberfläche, die in einer gegenüber ihrer Umrandung zurückversetzten Vertiefung angeordnet ist. In die Umrandung ist die Abdeckung 3 eingepasst, um mit ihrer Vorderseite einen homogenen, etwas konvex nach außen gewölbten Abschluss zu bilden. Die Vertiefung und damit im unbenutzten Zustand auch die Abdeckung 3 sind rundum von dem übrigen Bereich der Vorderseite des Gehäuses rahmenartig umgeben; auf der Unterseite des Rahmens fügt sich der Fußteil 4.1 der Halterung 4 an.

Im Übergangsbereich zwischen dem unteren oder alternativ dem oberen oder einem seitlichen Rahmenabschnitt des Gehäuses 2 und der Abdeckung 3 ist ein Griffmittel in Form einer Griffflasche 3.1 angeordnet, mit dem die eingesetzte Abdeckung 3 durch schräges Ziehen nach oben oder gegebenenfalls nach unten oder nach seitlich vorn, wie mit dem auf der Vorderseite der Abdeckung 3 aufgebrachten Informationsmittel in Form eines Informationspfeil 3.2 deutlich gemacht, schnell und einfach von dem Gehäuse 2 abgenommen werden kann.

Die Abdeckung 3 ist mittels eines oder mehrerer am oberen Randbereich angeformter Halteabschnitte 3.3 und eines oder mehrerer am unteren Rand der Abdeckung 3 angeformter weiterer Halteabschnitte 3.4 in daran angepassten, komplementären Gegenhalteelementen im Übergang der entsprechenden Rahmenabschnitte zu der Vertiefung am Gehäuse 2 fixiert. Denkbar sind auch andere, z.B. auf der Rückseite der Abdeckung 3 angeformte Halteabschnitte und in der Grundebene der Vertiefung entsprechend angeordnete Gegenhalteabschnitte. Die plattenartige, etwas konvex nach vorn gewölbte Abdeckung 3 aus Kunststoff hält infolge ihrer Elastizitätskräfte durch diese Schnappverbindung zuverlässig an dem Gehäuse und kann andererseits im Notfall leicht von diesem entfernt werden. Auch magnetische oder nach dem Prinzip eines Klettverschlusses wirksame Halteabschnitte und Gegenhalteelemente sind denkbar.

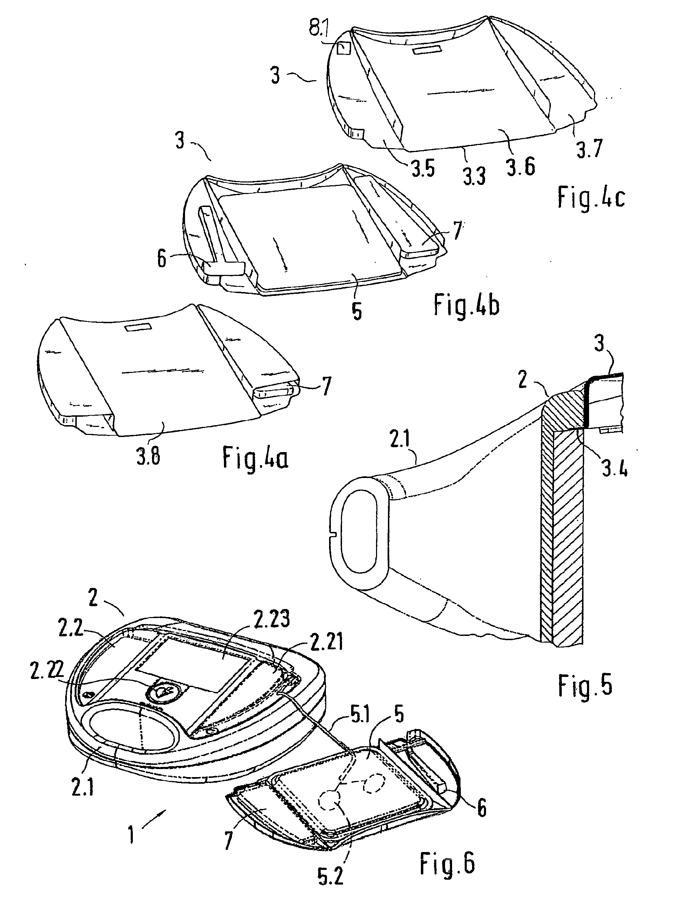

Es ist eine Sensoreinrichtung vorgesehen, mit der der Defibrillator 1 eingeschaltet wird, sobald die deckelartige Abdeckung 3 von dem Gehäuse 2 entfernt wird. Die Sensoreinrichtung weist beispielsweise eine magnetisch oder optoelektrisch oder elektromechanisch funktionierende Schaltvorrichtung auf, wobei ein an der Abdeckung 3 angeordnetes Sensorteil 8.1 (z.B. Permanentmagnet oder optisch wirksames Teil oder mechanisches Auslöseteil) mit einem an einer entsprechenden Gehäusestelle angeordneten Sensorelement 8.2 (z.B. Magnetfeld-Sensorelement, photoelektrisch ansprechendes Sensorelement oder mechanisch betätigbarer Schalter oder Taster) zusammenwirkt, wie aus den Fig. 3 und 4C ersichtlich. Dadurch erübrigt sich ein separater Ein-/Ausschalter und das Gerät ist nach richtiger Platzierung der Elektroden zum Auslösen des Defibrillationsimpulses bereit.

Auf der Rückseite der Abdeckung 3 sind mehrere Kammern ausgebildet, die durch angeformte Stege voneinander getrennt sind, wie Fig. 4c) erkennen lässt. In eine mittlere Kammer 3.6 sind Elektroden 5.2 (vgl. Fig. 6) eingelegt, die sich ihrerseits in einer vorzugsweise vakuumdicht oder zumindest staub- oder feuchtigkeitsdicht verschlossenen Elektrodenaufnahme 5 bzw. Verpackung befinden, wobei ein mit den Elektroden 5.2 verbundenes Verbindungskabel 5.1 abgedichtet aus der Elektrodenaufnahme 5 herausgeführt und mit seinem anderen Ende, an dem ein Stecker 5.3 angebracht ist, in eine Steckbuchse am Rand der Vertiefung eingesteckt ist. Die Steckbuchse ist mit einer im Innern des Gehäuses 2 angeordneten Elektronik des Defibrillators 1 elektrisch verbunden.

Seitlich von der mittleren Kammer 3.6 sind weitere Kammern 3.5 und 3.7 angeordnet, in denen geeignete Bedienungsgegenstände für die Benutzung des Defibrillators 1 aufgenommen sind, beispielsweise ein Rasierapparat 6, Handschuhe und eine Bürste 7, wie aus Fig. 4b) ersichtlich. Die Kammern 3.5, 3.6 und 3.7 mit den darin aufgenommenen Gegenständen sind auf der Innenseite mittels eines Abdeckteils 3.8 gemeinsam abgedeckt. Durch ein im oberen Bereich der Abdeckung 3 angeordnetes Fenster kann visuell von außen kontrolliert werden, ob die Kammer 3.6 z.B. nach einer Benutzung des Defibrillators 1 mit einer neuen Elektrodenpackung 5 bestückt ist. Die Grifflasche 3.1 kann beispielsweise an der Abdeckung 3 oder aber an der Elektrodenaufnahme 5 angeordnet sein, wobei sie zwischen dem unteren bzw. gegebenenfalls oberen oder seitlichen Rand der Abdeckung 3 und dem unteren Rahmenabschnitt des Gehäuses 2 hindurchgeführt ist. Bei Anbringung an der Elektrodenaufnahme 5 lässt sich damit ebenfalls kontrollieren, ob eine solche auf der Innenseite der Abdeckung 3 vorhanden ist. Außerdem hält der Benutzer beim Ziehen an der Griffflasche 3.1 bereits die Elektrodenpackung 5 mit den Elektroden in der Hand, so dass die schnelle Bedienung vereinfacht wird.

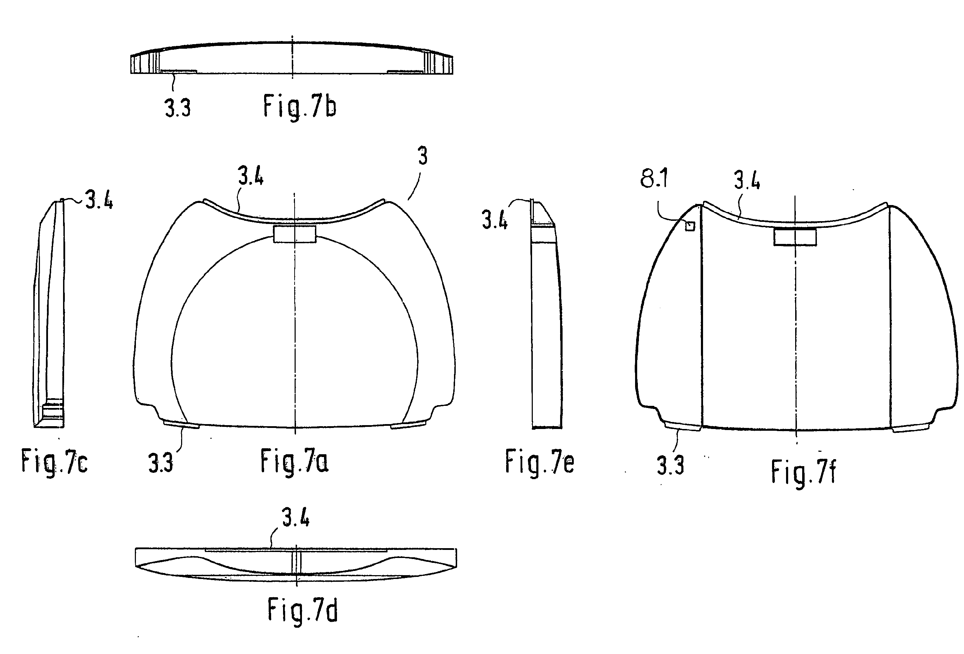

Der vergrößerte Ausschnitt nach Fig. 5 zeigt den am Rahmen des Gehäuses 2 eingesetzten Halteabschnitt 3.4 der Abdeckung 3. Fig. 7 zeigt in den sechs Ansichten der Teilbilder a) bis f) die Abdeckung 3 in Vorderansicht, Unteransicht, seitlicher Ansicht von rechts, von unten, seitlicher Ansicht von links sowie aus rückseitiger Ansicht, wobei die vorstehend bereits genannten Halteabschnitte 3.3 am unteren Rand und die weiteren Halteabschnitte 3.4 am oberen Rand deutlich erkennbar sind.

## Patentansprüche

1. Defibrillator (1) mit einer in einem Gehäuse (2) angeordneten Elektronik und daran anschließbaren, an einem Patienten anzulegenden Elektroden (5.2), wobei
die Elektroden (5.2) in einer auf der Innenseite einer aufklappbaren oder abnehmbaren Abdeckung (3) ausgebildeten Kammer (3.6) aufgenommen sind, **dadurch gekennzeichnet, dass** der Defibrillator (1) eine Sensoreinrichtung (8.1, 8.2) umfasst, die so ausgebildet ist, dass sie auf das Entfernen der Abdeckung (3) anspricht und mittels der der Defibrillator eingeschaltet wird, sobald die Abdeckung (3) von dem Gehäuse (2) entfernt wird.

2. Defibrillator nach Anspruch 1,
**dadurch gekennzeichnet,**
**dass** die Elektroden (5.2) auch im unbenutzten Zustand des Defibrillators (1) über ein Verbindungskabel (5.1) und einen damit verbundenen Stecker (5.3) an einer mit der Elektronik verbundenen Anschlussbuchse des Gehäuses (2) in einem von der Abdeckung im unbenutzten Zustand abgedeckten Hohlraum angeschlossen sind.

3. Defibrillator nach Anspruch 1 oder 2,
**dadurch gekennzeichnet,**
**dass** die Elektroden (5.2) mittels einer vakuumdichten, feuchtigkeitsdichte oder staubdichten Elektrodenaufnahme (5) in der Kammer (3.6) der Abdeckung (3) aufgenommen sind.

4. Defibrillator nach einem der vorhergehenden Ansprüche,
**dadurch gekennzeichnet,**
**dass** im Bereich der Außenseite der Abdeckung (3) ein von einem Benutzer erfassbares Griffmittel (3.1) angeordnet ist; mit dem die Abdeckung (3) von dem Gehäuse (2) wegreißbar ist.

5. Defibrillator nach Anspruch 4,
**dadurch gekennzeichnet,**
**dass** das Griffmittel eine mit der Abdeckung (3) oder mit der Elektrodenaufnahme (5) verbundene Grifflasche (3.1) ist.

6. Defibrillator nach einem der vorhergehenden Ansprüche,
**dadurch gekennzeichnet,**
**dass** die Abdeckung (3) mit Haltemitteln (3.3, 3.4) versehen ist, die zum Fixieren an dem Gehäuse (2) in an diesem angeordneten Gegenhalteelementen eingerastet, eingeklipst oder eingeschnappt sind oder daran magnetisch gehalten sind.

7. Defibrillator nach einem der vorhergehenden Ansprüche,
**dadurch gekennzeichnet,**
**dass** auf der Innenseite der Abdeckung (3) weitere Kammern (3.6, 3.7) ausgebildet und weitere herausnehmbare Bediengegenstände (6, 7) aufgenommen sind.

8. Defibrillator nach einem der vorhergehenden Ansprüche,
**dadurch gekennzeichnet,**
**dass** die mindestens eine Kammer (3.5, 3.6, 3.7) mit den Elektroden (5.2) und gegebenenfalls weiteren Bediengegenständen (6, 7) mittels eines abnehmbaren inneren Abdeckteils (3.8) abgedeckt ist.

9. Defibrillator nach einem der vorgehenden Ansprüche,
**dadurch gekennzeichnet,**
**dass** eine von der Abdeckung (3) im nicht benutzten Zustand abgedeckte und im Benutzungszustand freigegebene frontseitige Gehäusewandung als Bedienfeld (2.2) mit mindestens einem Auslöseelement (2.22) für die Defibrillation und Benutzerführungselementen (2.22, 2.21) ausgebildet ist.

10. Defibrillator nach einem der Ansprüche 4 bis 9,
**dadurch gekennzeichnet,**
**dass** auf der Außenseite der Abdeckung (3) ein Informationsmittel (3.2) zum Betätigen des Griffmittels (3.1) angeordnet ist.

## Claims

1. Defibrillator (1) having an electronic device, which is disposed in a housing (2), and electrodes (5.2), which are connectable thereto and can be applied to a patient, the electrodes (5.2) being accommodated in a chamber (3.6) provided on the inside of a cover (3), which can be flipped-open or removed, **characterised in that** the defibrillator (1) includes a sensor arrangement (8.1, 8.2), which is designed so that it responds to the removal of the cover (3), and by means of which the defibrillator is switched-on as soon as the cover (3) is removed from the housing (2)

2. Defibrillator according to claim 1, **characterised in that**, even in the unused state of the defibrillator (1), the electrodes (5.2) are connected, via a connecting cable (5.1) and a plug (5.3) connected thereto, to a connection socket of the housing (2), which connection socket is connected to the electronic device, in a cavity which is covered, in the unused state, by the cover.

3. Defibrillator according to claim 1 or 2, **characterised in that** the electrodes (5.2) are accommodated in the chamber (3.6) of the cover (3) by means of a vacuum-sealed, moisture-proof or dust-proof electrode receptacle (5).

4. Defibrillator according to one of the preceding claims, **characterised in that** a handle (3.1) is disposed in the region of the outside of the cover (3), which handle can be grasped by a user, and with which the cover (3) can be torn away from the housing (2).

5. Defibrillator according to claim 4, **characterised in that** the handle is a gripping tab (3.1) which is connected to the cover (3) or to the electrode receptacle (5).

6. Defibrillator according to one of the preceding claims, **characterised in that** the cover (3) is provided with retaining means (3.3, 3.4) which, for fixing on the housing (2), are locked, clipped or snapped into counter-retaining elements, disposed on said housing, or are magnetically retained thereon.

7. Defibrillator according to one of the preceding claims, **characterised in that**, on the inside of the cover (3), additional chambers (3.6, 3.7) are provided and additional removable operating items (6, 7) are accommodated.

8. Defibrillator according to one of the preceding claims, **characterised in that** the at least one chamber (3.5, 3.6, 3.7), having the electrodes (5.2) and possibly additional operating items (6, 7), is covered by means of a removable inner covering part (3.8).

9. Defibrillator according to one of the preceding claims, **characterised in that** a housing wall at the front, which in the unused state is covered by the cover (3) and in the used state is exposed, is in the form of a control panel (2.2) with at least one triggering element (2.22) for the defibrillation and user guide elements (2.22, 2.21).

10. Defibrillator according to one of claims 4 to 9, **characterised in that** an information means (3.2) for actuating the handle (3.1) is disposed on the outside of the cover (3).

## Revendications

1. Défibrillateur (1) avec une électronique disposée dans un boîtier (2) et avec des électrodes (5.2) à appliquer sur un patient pouvant y être raccordées, les électrodes (5.2) étant logées dans une chambre (3.6) formée du côté intérieur d'un couvercle (3) relevable ou amovible,
**caractérisé en ce**
**que** le défibrillateur (1) comprend un dispositif capteur (8.1, 8.2) qui est conçu de manière à réagir à l'enlèvement du couvercle (3) et au moyen duquel le défibrillateur est mis en service dès que le couvercle (3) est enlevé du boîtier (2).

2. Défibrillateur selon la revendication 1,
**caractérisé en ce**
**que** les électrodes (5.2) sont raccordées même dans l'état non utilisé du défibrillateur (1) par un câble de jonction (5.1) et un connecteur (5.3) relié à ce dernier à une douille de raccordement du boîtier (2) reliée à l'électronique dans une cavité recouverte par le couvercle dans l'état non utilisé.

3. Défibrillateur selon la revendication 1 ou 2,
**caractérisé en ce**
**que** les électrodes (5.2) sont logées au moyen d'un porte-électrodes (5) étanche au vide, étanche à l'humidité ou étanche aux poussières dans la chambre (3.6) du couvercle (3).

4. Défibrillateur selon l'une des revendications précédentes,
**caractérisé en ce**
**qu'**un moyen de préhension (3.1) pouvant être saisi par un utilisateur, avec lequel le couvercle (3) peut être arraché du boîtier (2), est disposé dans la région du côté extérieur du couvercle (3).

5. Défibrillateur selon la revendication 4,
**caractérisé en ce**
**que** le moyen de préhension est une languette de préhension (3.1) reliée au couvercle (3) ou au porte-électrodes (5).

6. Défibrillateur selon l'une des revendications précédentes,
**caractérisé en ce**
**que** le couvercle (3) est muni de moyens de maintien (3.3, 3.4) servant à sa fixation au boîtier (2), qui sont encliquetés, clipsés ou enclenchés dans des éléments de maintien complémentaires disposés sur celui-ci ou maintenus magnétiquement sur celui-ci.

7. Défibrillateur selon l'une des revendications précédentes,
**caractérisé en ce**
**que** d'autres chambres (3.6, 3.7) sont formées du côté intérieur du couvercle (3), dans lesquelles d'autres objets de service amovibles (6, 7) sont logés.

8. Défibrillateur selon l'une des revendications précédentes,
**caractérisé en ce**
**que** ladite au moins une chambre (3.5, 3.6, 3.7) contenant les électrodes (5.2) et le cas échéant d'autres objets de service (6, 7) est recouverte au moyen d'une partie de couvercle intérieure (3.8) amovible.

9. Défibrillateur selon l'une des revendications précédentes,
**caractérisé en ce**
**qu'**une paroi frontale de boîtier recouverte par le couvercle (3) dans l'état non utilisé et dégagée dans l'état d'utilisation est conçue comme panneau de commande (2.2) avec au moins un élément de déclenchement (2.22) pour la défibrillation et des éléments de guidage de l'utilisateur (2.22, 2.21).

10. Défibrillateur selon l'une des revendications 4 à 9,
**caractérisé en ce**
**qu'**un moyen d'information (3.2) concernant la manoeuvre du moyen de préhension (3.1) est disposé du côté extérieur du couvercle (3).
